Europäisches Patentamt

European Patent Office

Office européen des brevets

(11) Veröffentlichungsnummer: **0 086 738**
**A1**

(12) # EUROPÄISCHE PATENTANMELDUNG

(21) Anmeldenummer: 83730016.9

(22) Anmeldetag: **10.02.83**

(51) Int. Cl.³: **A 61 L 2/10**, C 02 F 1/32

(30) Priorität: **13.02.82 DE 3205524**

(43) Veröffentlichungstag der Anmeldung: **24.08.83**
**Patentblatt 83/34**

(84) Benannte Vertragsstaaten: **AT BE CH DE FR GB IT LI LU NL SE**

(71) Anmelder: **Horstmann, Georg, Geibelstrasse 2, D-4902 Bad Salzuflen (DE)**

(72) Erfinder: **Horstmann, Georg, Geibelstrasse 2, D-4902 Bad Salzuflen (DE)**

(74) Vertreter: **Thömen, Uwe, Dipl.-Ing., Patentanwalt U. Thömen Zeppelinstrasse 5, D-3000 Hannover (DE)**

(54) **Vorrichtung zum Entkeimen von Flüssigkeiten oder Gasen.**

(57) Neben den Rundstrahlern, die ein sich kreisförmig erstreckendes polares Strahlungsfeld erzeugen, werden zum Entkeimen von Flüssigkeiten oder Gasen auch Flachstrahler eingesetzt, weil sich mit diesen eine erheblich höhere Strahlungsintensität erzeugen lässt. Allerdings lässt sich mit den Flachstrahlern kein polares Feld erzeugen. Vielmehr besitzen die Flachstrahler an ihren beiden gegenüberliegenden breiten Längsseiten jeweils ein paralleles Strahlungsfeld, während an den gegenüberliegenden Schmalseiten nur eine geringe Strahlung auftritt. Deshalb konnten die Flachbrenner bisher nicht bei solchen Vorrichtungen eingesetzt werden, die ein polares Strahlungsfeld zum Entkeimen erfordern.

Durch die Erfindung wird hier Abhilfe geschaffen, indem der Flachstrahler durch einen Motor angetrieben und in Umdrehungen um die Flachstrahler-Längsachse versetzt wird. Die an den beiden Längsseiten des Flachstrahlers austretenden parallelen Strahlen hoher Strahlungsintensität erfassen somit nacheinander alle Bereiche zwischen 0 und 360°, so dass quasi ein polares Feld wie bei einem Rundstrahler erzeugt wird.

Vorrichtung zum Entkeimen von
Flüssigkeiten oder Gasen

Die Erfindung betrifft eine Vorrichtung zum Entkeimen von Flüssigkeiten oder Gasen mittels ultravioletter
(UV-) Strahlung, mit einem von dem zu entkeimenden Medium umgebenen Hüllrohr, in welchem sich eine in ihrem Querschnitt
von der Kreisform abweichende und mit elektrischen Anschlüssen
versehene UV-Brennerlampe, insbesondere ein als Quecksilber-
Niederdrucklampe ausgebildeter Flachstrahler, befindet.

Bekanntlich gewinnt die Durchführung von photochemischen Reaktionen mit Hilfe von UV-Strahlung eine zunehmende Bedeutung, wobei photochemische Entkeimungs- und Sterilisierungsvorgänge ein besonders wichtiges Gebiet darstellen.
Die photochemischen Entkeimungs- und Sterilisierungsvorgänge
zeigen dabei eine ausgeprägte Abhängigkeit von der Intensität
der UV-Strahlung, so daß es entscheidend auf eine möglichst
hohe Strahlungsintensität ankommt. Dies ist darauf zurückzuführen, daß die betreffenden Reaktionen eine gleichzeitige Anwesenheit einer bestimmten Mindestzahl von (z.B. 4 oder 5)
Lichtquanten an der zu reagierenden Molekülstelle benötigen
und deshalb bei Anwendung einer Strahlung geringer Intensität,
welche diese Mindestzahl von Lichtquanten nicht gleichzeitig
zur Verfügung zu stellen vermag, nicht ablaufen können. Dem
kann dann beispielsweise auch nicht durch eine Verlängerung
der Bestrahlungsdauer abgeholfen werden.

Durch die DE-OS 28 25 018 ist zum Entkeimen von
Flüssigkeiten oder Gasen mittels UV-Strahlung schon eine UV-
Brennerlampe bekannt geworden, welche das Erfordernis der
relativ hohen Strahlungsintensität bestens erfüllt. Es handelt sich dabei um eine in ihrem Querschnitt von der Kreisform
abweichende UV-Brennerlampe, die einen länglich-flachen Querschnitt besitzt und inzwischen in der Fachwelt als sogenannter Flachstrahler bezeichnet wird. Während die zuvor benutzten
UV-Brennerlampen als Rundstrahler stets kreisförmig ausgebildet waren, hat sich in der Praxis gezeigt, daß sich mit den
Flachstrahlern eine erheblich größere Strahlungsdichte erzielen läßt, da das innerhalb der Brennerlampe befindliche leuchtende Plasma dabei die Form eines "Brettes", also eines Körpers
mit großer Breite und nur geringer Tiefe, hat. Auch lassen sich
die Flachbrenner mit viel höherer Energieaufnahme als bisher
betreiben, wodurch die Strahlungsdichte zusätzlich in erheblichem Umfang erhöht wird.

Bei der Verwendung der Flachstrahler ist allerdings
zu berücksichtigen, daß sie ihre Strahlung im wesentlichen nur
an den beiden gegenüberliegenden Breitseiten - dort mit außerordentlich hoher Strahlungsdichte - abgeben. Es liegt also keine
polare Abstrahlung vor, bei der gleichmäßig um die UV-Brennerlampe herum verteilt UV-Strahlung anzutreffen ist. Vielmehr
gibt es an den beiden Schmalseiten des Flachstrahlers Bereiche,
in denen kaum eine UV-Strahlung vorliegt. Dadurch wird aber
die Verwendung des an sich so enorm vorteilhaften Flachstrahlers eingeengt, da sich eine Entkeimung bzw. Sterilisation von
Flüssigkeiten oder Gasen am einfachsten mit einem polaren Strahlungsfeld verwirklichen läßt.

Hier setzt die Erfindung ein, der die Aufgabe zugrunde liegt, den Verwendungsbereich der in der Praxis so vorteilhaften Flachstrahler zu erweitern und eine Vorrichtung zu
schaffen, welche es ermöglicht, auch mit Flachstrahlern quasi
ein homogenes polares Strahlungsfeld zu erzeugen.

Zur Lösung dieser Aufgabe sieht die Erfindung bei
der im Oberbegriff des Anspruchs 1 vorausgesetzten Vorrichtung

0086738

vor, daß relativ zwischen der UV-Brennerlampe und dem zu
entkeimenden Medium eine Drehbewegung erfolgt, so daß die
UV-Strahlen nacheinander auf unterschiedliche Bereiche des
zu entkeimenden Mediums einwirken. Vorzugsweise wird eine
relative Bewegung zwischen dem zu entkeimenden Medium und
der UV-Brennerlampe dadurch erzeugt, daß letztere um ihre
Längsachse drehbar in dem Hüllrohr angeordnet ist, und daß
die elektrischen Anschlüsse der UV-Brennerlampe durch
Schleifringe an ihrem Sockel gebildet sind, wobei die Stromzufuhr über Bürsten erfolgt, die in Kontakt mit den Schleifringen stehen.

Durch die Erfindung wird der Anwendungsbereich
der vorteilhaften Flachstrahler erheblich erweitert, da es
nunmehr möglich ist, mit einem Flachstrahler quasi ein polares Feld bzw. eine polare Abstrahlung zu erzeugen, während
die Verwendung von Flachstrahlern bisher auf solche Fälle beschränkt war, in denen an den beiden Längsseiten des Flachstrahlers eine parallele Abstrahlung erwünscht war.

Durch die Drehung des Flachstrahlers um die Längsachse werden nacheinander über einen Winkel von 360° alle Bereiche des zu entkeimenden Mediums mit der hohen Strahlungsintensität des Flachbrenners angestrahlt. Daneben besitzt die
Erfindung aber noch einen weiteren Vorteil. Infolge der Drehung
des Flachstrahlers innerhalb des runden Hüllrohres entsteht
nämlich eine gewisse Zirkulation innerhalb des Hüllrohres,
und diese Zirkulation ermöglicht in gewissem Grade eine Kühlung des Flachstrahlers, so daß dieser mit relativ hoher Leistung betrieben werden kann.

Für die Erzeugung der Drehbewegung für den Flachstrahler kann ein Kleinstmotor verwendet werden, der auf einem Deckel angeordnet ist, mit welchem der Reaktionsraum für das zu entkeimende Medium abgeschlossen ist. Die Anzahl der Umdrehungen in einer bestimmten Zeiteinheit wird zweckmäßigerweise so gewählt, daß eine Drehung von 180° erfolgt ist, bevor das Medium den Weg längs des Flachstrahlers passiert hat. Auf diese Weise ist sichergestellt, daß das polare Strahlungsfeld alle Bereiche des zu entkeimenden Mediums erfaßt.

Andere vorteilhafte Weiterbildungen der Erfindung sind in den Unteransprüchen angegeben.

Anhand des in der Zeichnung dargestellten Ausführungsbeispiels wird die Erfindung nachfolgend näher erläutert. Es zeigen:

Fig. 1    eine schematische Querschnittsansicht einer Vorrichtung mit einem drehbaren Flachstrahler,

Fig. 2    eine Querschnittsansicht des Flachstrahlers selbst, und

Fig. 3    eine andere Ausführungsform.

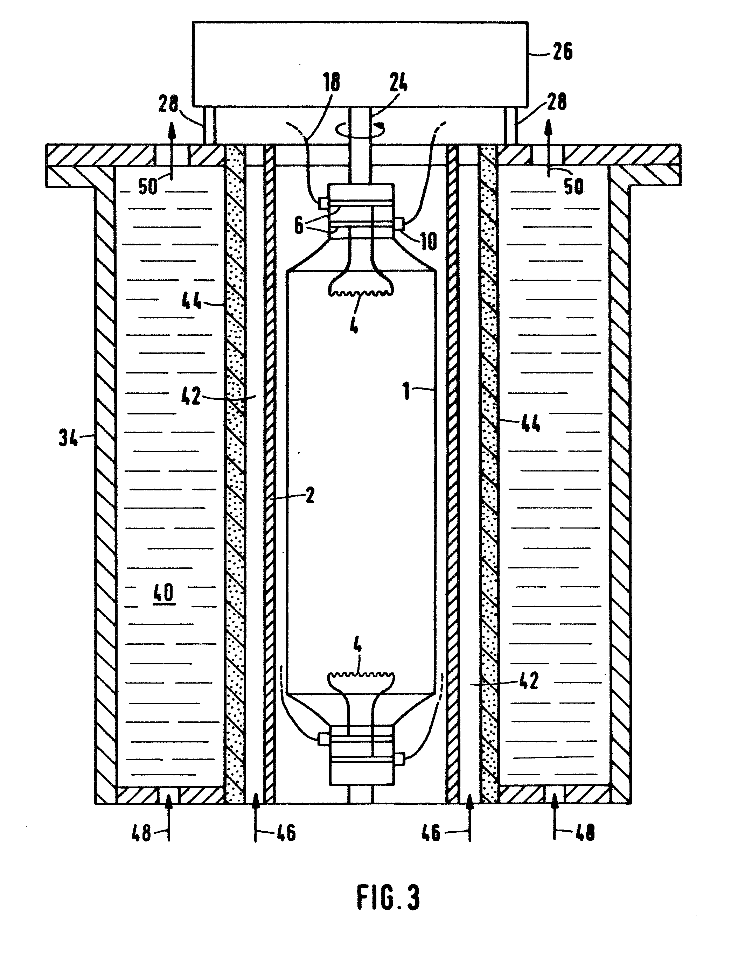

Die zeichnerisch dargestellte Vorrichtung zum Entkeimen von Flüssigkeiten oder Gasen mittels UV-Strahlung umfaßt eine UV-Brennerlampe, die als Flachstrahler 1 ausgebildet ist. Wie die Querschnittsansicht in Fig. 2 verdeutlicht, erzeugt ein solcher Flachstrahler an seinen beiden gegenüberliegenden Längsseiten jeweils ein paralleles Strahlungsfeld.

Der Flachstrahler 1, der innerhalb eines Hüllrohres 2 angeordnet ist, besitzt an seinen beiden gegenüberliegenden äußeren Enden je eine Elektrode 4, deren Anschlüsse zu Schleifringen 6 bzw. 8 führen. Um trotz der Drehung des Flachstrahlers 1 eine Stromzufuhr zu ermöglichen, sind - wie bei Elektromotoren

0086738

bekannt - für jeden Schleifring Bürsten 10 und 12 vorgesehen, von denen Anschlußleitungen 18 und 20 zur elektrischen Versorgung führen.

Der obere Sockel 14 des Flachstrahlers 1 steht über eine Welle 24 in Wirkverbindung mit einem kleinen Motor 26, welcher mittels Befestigungsstützen 28 auf einem Deckel 32 gehalten ist. Mit diesem Deckel wird das Reaktorgehäuse 34 unter Verwendung eines Dichtungsringes 30 abgeschlossen.

Mit dem unteren Sockel 16 ist der Flachstrahler 1 drehbar in einem Sockellager 22 gelagert, welches sich am unteren Ende des Hüllrohres 2 befindet. Das Hüllrohr 2 besteht aus Quarz, welches für die von dem Flachstrahler 1 erzeugte UV-Strahlung durchlässig ist. Um ein Drehen - vgl. den Pfeil A - des Flachstrahlers 1 zu ermöglichen, ist das Hüllrohr 2 im Querschnitt gesehen kreisrund ausgebildet. Diese Kreisform ist besonders vorteilhaft, weil das Hüllrohr 2 so der Druckbelastung durch das umgebende Medium - hier Wasser 40 - am besten standhält.

Die gezeigte Vorrichtung besitzt noch einen Einlauf 36, durch welchen das zu entkeimende Wasser 40 in das Reaktorgehäuse eintreten und das eingetauchte Hüllrohr 2 umspülen kann. Durch einen Auslauf 38 fließt das entkeimte Wasser ab.

Es ist ersichtlich, daß bei stillstehendem Flachstrahler nur an den beiden gegenüberliegenden Längsseiten die UV-Strahlung vorhanden ist, so daß dann nicht alle Bereiche des das Hüllrohr 2 umspülenden Wassers entkeimt werden. Infolge der Drehung des Flachstrahlers überstreichen die erzeugten Strahlungsfelder jedoch nacheinander alle Bereiche des Reaktionsraumes. Es stellt sich somit die gleiche Wirkung ein, als wenn in dem Hüllrohr ein Rundstrahler mit einem polaren Strahlungsfeld angeordnet wäre. Wie schon gesagt, lassen sich allerdings mit dem Flachstrahler erheblich größere Strahlungsdichten erzielen, und in Versuchen ist festgestellt worden, daß mit der erfindungsgemäßen Vorrichtung unter Verwendung nur eines einzigen Flachstrahlers 30 bis 40 m³/h entkeimt werden können. Solche hohen Durchsatzmengen konnten bisher mit den herkömmlichen Vorrichtungen nicht realisiert werden.

Das zu entkeimende Wasser bewegt sich vom Einlaß 36 längs des Flachstrahlers 1 zum unteren Auslaß 38. Die Anzahl der Umdrehungen pro Minute des Flachstrahlers 1 wird so gewählt, daß innerhalb derjenigen Zeit, in welcher das Wasser 40 den Weg längs des Flachstrahlers 1 zurückgelegt hat, mindestens eine halbe Umdrehung des Flachstrahlers 1 erfolgt. Dadurch ist gewährleistet, daß alle Wasserteilchen vor dem Verlassen des Reaktorgehäuses 34 der hohen Strahlungsintensität des Flachstrahlers 1 unterworfen sind.

In dem voranstehend beschriebenen Ausführungsbeispiel der Erfindung wurde ein fortwährendes Drehen des Flachstrahlers 1 angenommen. Allerdings ist die Erfindung hierauf nicht beschränkt. Vielmehr läßt sich die angestrebte Wirkung auch dadurch erreichen, daß der Flachstrahler 1 oszillierend hin und her bewegt wird. So kann der Flachstrahler 1 zunächst eine Linksdrehung und daran anschließend eine entgegengestzt gerichtete Rechtsdrehung ausführen. Dabei umfaßt die Drehbewegung in jeweils der einen Richtung mindestens 180°, so daß auch bei dieser oszillierenden Drehbewegung gewährleistet ist, daß alle Bereiche des zu entkeimenden Wassers 40 der Strahlung des Flachstrahlers 1 ausgesetzt sind.

Für die untere Lagerung des Flachstrahlers 1 läßt sich bei Anwendung der oszillierenden Drehbewegung in vorteilhafter Weise eine Spiralfeder einsetzen, wie sie von der Unruhe bei einem Uhrwerk her bekannt ist.

Bei der Verwendung von Flachstrahlern spielt der Gesichtspunkt einer hinreichenden Kühlung eine entscheidende Rolle, und es wurde voranstehend schon erwähnt, daß infolge der durch die Drehbewegung des Flachstrahlers 1 hervorgerufenen Zirkulation bereits ein gewisser Kühleffekt erreicht wird. Dieser läßt sich bei der Erfindung weiterhin dadurch verbessern, daß in das Hüllrohr 2 Kühlluft oder ein anderes Kühlmedium eingeleitet wird.

Eine besonders vorteilhafte Art der Kühlung ist in Fig. 3 dargestellt, welche einen Reaktor zeigt, bei dem im Abstand um das Hüllrohr 2 herum ein Kühlrohr 44 aus Quarz angeordnet ist. Dadurch entsteht zwischen dem Hüllrohr 2 und dem Kühlrohr 44 ein Ringraum, der als Kühlkammer 42 verwendet wird.

Durch den Pfeil 46 ist angedeutet, daß in die Kühlkammer 42 ein Kühlmedium eingeführt wird, welches am oberen Ende des Reaktors wieder aus der Kühlkammer 42 austritt. Als Kühlmedium läßt sich in vorteilhafter Weise Kühlluft verwenden, die unten eingeblasen und oben abgeführt wird. Es ist aber auch die Verwendung von flüssigem Kühlmittel möglich.

Damit die UV-Strahlen des Flachstrahlers 1 in den Reaktionsraum gelangen können, in welchem sich das zu entkeimende Wasser 40 befindet, besteht das Kühlrohr aus Quarz, wobei die Wandstärke etwa 4 bis 6 mm betragen kann. Eine besonders wirksame Kühlung ergibt sich, wenn sowohl in das Hüllrohr 2 als auch in die Kühlkammer 42 Kühlmedium geführt wird.

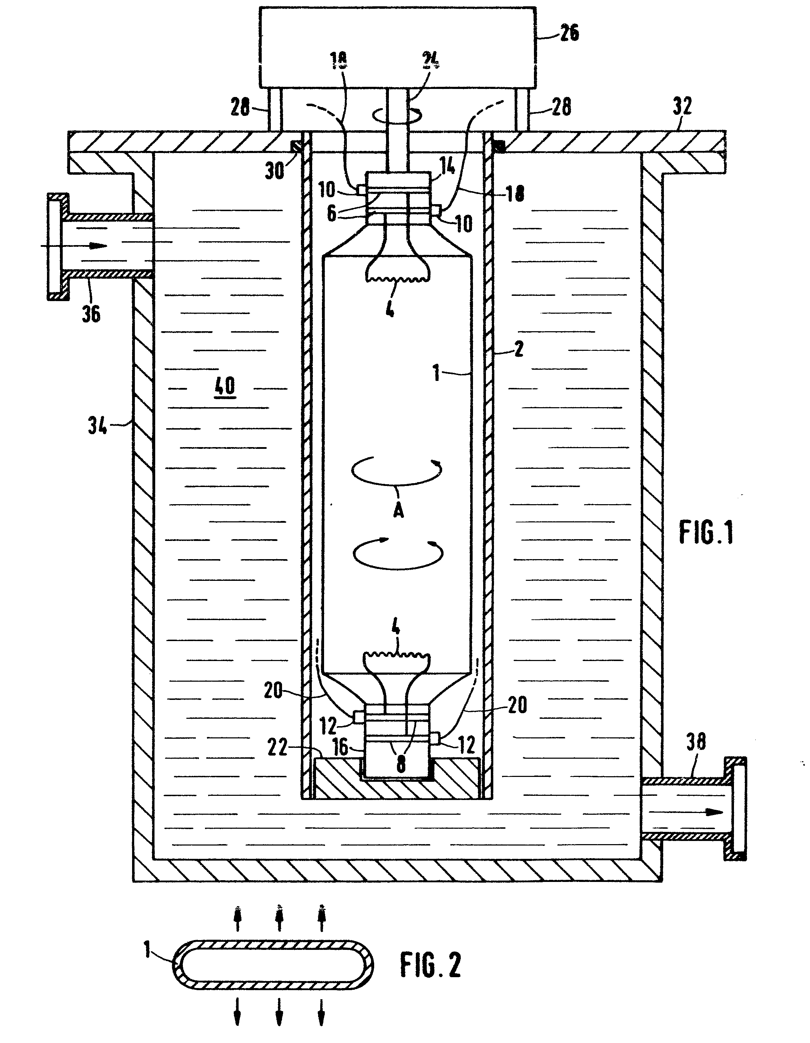

Abweichend von der in Fig. 1 gezeigten Ausführungsform wird bei dem Reaktor gemäß Fig. 3 das zu entkeimende Wasser von unten her zugeführt, was in der schematischen Darstellung durch die beiden Pfeile 48 angedeutet ist. Die Pfeile 50 verdeutlichen, daß das entkeimte Wasser am oberen Ende des Reaktors abgeführt wird.

Georg Horstmann                          375/11 EU


P a t e n t a n s p r ü c h e


1.      Vorrichtung zum Entkeimen von Flüssigkeiten oder
Gasen mittels ultravioletter (UV-)Strahlung, mit einem von
dem zu entkeimenden Medium umgebenden Hüllrohr, in welchem
sich eine in ihrem Querschnitt von der Kreisform abweichende und mit elektrischen Anschlüssen versehene UV-Brennerlampe, insbesondere ein als Quecksilber-Niederdrucklampe ausgebildeter Flachstrahler, befindet, dadurch gekennzeichnet,
daß relativ zwischen der UV-Brennerlampe (1) und dem zu entkeimenden Medium (40) eine Drehbewegung (A) erfolgt, so daß
die UV-Strahlen nacheinander auf unterschiedliche Bereiche
des zu entkeimenden Mediums (40) einwirken.

2.      Vorrichtung nach Anspruch 1, dadurch gekennzeichnet,
daß die UV-Brennerlampe (1) um ihre Längsachse drehbar in dem
Hüllrohr (2) angeordnet ist, daß die elektrischen Anschlüsse
durch Schleifringe (6, 8) am Sockel (14, 16) der UV-Brennerlampe (1) gebildet sind und daß die Stromzufuhr über Bürsten (10, 12) erfolgt, die in Kontakt mit den Schleifringen
(6, 8) stehen.

3.      Vorrichtung nach Anspruch 2, dadurch gekennzeichnet, daß die UV-Brennerlampe (1) von einem außerhalb des Hüllrohres (2) angeordneten Motor (26) angetrieben ist.

4.      Vorrichtung nach einem der vorhergehenden Ansprüche
2 und 3, dadurch gekennzeichnet, daß die Zuführung (18, 20)
für die Bürsten (10, 12) durch einen Deckel (32) geführt ist,
mit welchem ein das Medium (40) aufnehmender Reaktionsraum
abgeschlossen ist.

5.      Vorrichtung nach einem der vorhergehenden Ansprüche
2 bis 4, dadurch gekennzeichnet, daß eine Antriebswelle (24)
des Motors (26), die in Wirkverbindung mit der UV-Brennerlampe (1) steht, durch eine Öffnung des Deckels (32) geführt
ist.

6.      Vorrichtung nach einem der vorhergehenden Ansprüche
3 bis 5, dadurch gekennzeichnet, daß der Motor (26) durch den
Deckel (32) gehalten ist.

7.      Vorrichtung nach einem der vorhergehenden Ansprüche
2 bis 6, dadurch gekennzeichnet, daß die UV-Brennerlampe (1)
an ihrem dem Motor (26) abgewandten Ende drehbar in dem Hüllrohr (2) gelagert (22) ist.

8.      Vorrichtung nach einem der vorhergehenden Ansprüche
1 bis 7, dadurch gekennzeichnet, daß die UV-Brennerlampe (1)
oszillierend um ihre Längsachse hin und her bewegt wird.

9.      Vorrichtung nach Anspruch 8, dadurch gekennzeichnet, daß die Drehbewegungen mindestens einen Winkel von 180° umfassen.

10.     Vorrichtung nach einem der vorhergehenden Ansprüche 1 bis 9, dadurch gekennzeichnet, daß um das Hüllrohr (2) im Abstand ein Kühlrohr (44) aus einem für UV-Strahlung durchlässigen Material angeordnet ist, wodurch zwischen dem Hüllrohr (2) und dem Kühlrohr (44) eine Kühlkammer (42) nach Art eines Ringraumes gebildet wird.

FIG.1

FIG.2

FIG.3

Europäisches
Patentamt

**EUROPÄISCHER RECHERCHENBERICHT**

0086738
Nummer der Anmeldung

EP 83 73 0016

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| D,A | DE-A-2 825 018 (GEORG HORSTAMNN) * Ansprüche * ----- | 1 | A 61 L 2/10 C 02 F 1/32 |
|  |  |  | **RECHERCHIERTE SACHGEBIETE (Int. Cl. ³)** |
|  |  |  | A 61 L 2/10 C 02 F 1/32 |

Der vorliegende Recherchenbericht wurde für alle Patentansprüche erstellt.

| Recherchenort DEN HAAG | Abschlußdatum der Recherche 28-04-1983 | Prüfer MALHERBE Y.J.M. |
|---|---|---|

EPA Form 1503. 03.82

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument